# EUROPEAN PATENT SPECIFICATION

(11) **EP 2 609 837 B1**
(45) Date of publication and mention of the grant of the patent: **12.09.2018**
(21) Application number: 12199800.9
(22) Date of filing: 02.02.2009
(51) Int. Cl.: A47G 9/02, A61F 7/00, A61F 7/03

(54) **Warming blanket and method of fabricating the same**
Wärmdecken und Verfahren zu deren Herstellung
Couverture chauffante et son procédé de fabrication

(30) Priority: 03.06.2008 US 58283 P; 13.06.2008 US 61404 P
(43) Date of publication of application: 03.07.2013
(62) Divisional of application: 09758799.2
(73) Proprietor: Apricity LLC, Alpine, UT 84004 (US)
(72) Inventor: Vergona, Joseph Blase, Suwanee, GA 30024 (US); Kyker, Mark Uel, Carmel, IN 46032 (US)
(74) Representative: Phillips & Leigh LLP

(56) References cited:
- WO-A-03/000564
- GB-A- 2 401 055
- US-A- 2 596 547
- US-A- 4 969 459
- US-A- 5 918 333
- US-A1- 2006 057 917
- US-A1- 2006 135 016
- US-A1- 2007 284 356

## Description

### RELATED APPLICATIONS

This application claims priority to both U.S. Serial No. 61/058,283, entitled "METHOD AND APPARATUS FOR PATIENT WARMING", filed June 3, 2008; and to U.S. Serial No. 61/061,404, entitled "WARMING BLANKETS AND METHODS OF FABRICATING THE SAME", filed June 13, 2008.

### FIELD OF THE INVENTION

Embodiments of the invention relate generally to warming blankets and methods of fabricating and using the same.

### BACKGROUND OF THE INVENTION

Conventional blankets are utilized in association with a wide variety of different applications and activities, for instance, recreational uses, outdoor uses, medical uses, etc. Such blankets are typically utilized in order to provide heat and/or protection to a user or group of users. A warming blanket is known from US 2596547. In some instances, one or more electrical heating elements can be incorporated into such blankets in order to provide additional heat or comfort. These electrical heating elements are typically powered by an electrical power source. The portability of such blankets is often limited when the electrical heating elements are activated. In some instances, certain conventional blankets did not provide an adequate level of comfort in certain situations due to bulk or lack of performance as some users, such as medical patients, complained of such deficiencies. Additionally, safety considerations can arise when using such blankets due to environmental conditions including the presence of moisture or due to electromagnetic fields generated by such blankets. Therefore, it is often not feasible to utilize these conventional blankets in certain environments or in certain applications.

In some circumstances, certain persons have a need to maintain or otherwise regulate their body warmth. Humans have a very tight control of thermoregulation with the ability to maintain within a thermoregulatory threshold of approximately 0.2 °C to maintain a targeted thermal value. This tight threshold may be reduced in the elderly or the sick. The primary autonomic defenses of thermal challenge are sweating and thermoregulatory vasoconstriction that occurs primarily in the distal extremities, such as fingers and toes. This shunting of blood allows the core or central temperature to maintain a temperature of approximately 3°C to approximately 4°C warmer than the periphery (arms and legs). Shivering is the last autonomic defense that generates approximately 2 times to approximately 3 times the normal metabolic thermal production.

On induction of general anesthesia or major conduction anesthesia the normal thermal preventive measures are inhibited. The thermoregulation interthreshold (typically about 0.2 °C) range is widened by approximately 20 times so as to prevent the maintenance of normothermia. Obviously all behavioral modifications are prevented with anesthesia and the resultant loss of tonic vasoconstriction in the fingers and toes causes the redistribution of heat from the core to the periphery at a loss of approximately 0.6 °C per hour for about the first two hours. Approximately 90 percent of all heat is lost via the skin surface due to the vasodilation with only a small amount of loss via radiant and convective means. This loss will continue after the first two hours but at a reduced rate, primarily from continued heat loss pathways that exceed heat production as the metabolic rate is decreased under anesthesia. Eventually, reestablishment of thermoregulation will occur but only after significant hypothermia with the below mentioned comorbidities.

One conventional method to warm a hypothermic (below about 36°C) patient intraoperatively is with a forced air warmer. Use of blankets, convective covers, heated water mattresses, and fluid warming generally do not result in significant warming of an anesthetized patient.

Maintenance of normothermia is of clinical importance as well as patient satisfaction and may have an effect on payment for services for both the practitioner and the facility. Conventional active warming devices can include a forced air warmer that consists of a stand alone machine that has an electrical cord, corrugated tubing that affixes to the plastic disposable sheet that is placed over the patient in the operating room. The controls can be placed on a separate device that generates the warm flow or air that blows through the sheet. Most hospitals and surgery centers utilize this only in the operating room as it can require multiple machines to provide flow in each patient care space. The device itself can be somewhat cumbersome, and has cords that are intrusive especially in the preoperative and post operative site. At some or all settings the forced air warmer produces significant noise within the environment.

Patients undergoing anesthesia, general, neuraxial and even sedation will experience varying degrees of hypothermia (becoming cold). This effect is most pronounced during about the first two hours of anesthesia. Typically this loss of core temperature occurs at about 0.6°C per hour for about the first two hours. This resulting unintentional anesthetic induced mild hypothermia has many well documented ill effects, some of which include an increase in surgical wound infections, coagulopathy, increased cardiac morbidity, prolonged emergence and recovery translating into longer hospital stays and increased costs.

In fact, maintenance of normothermia is one of the proposed performance measures put forth by the American Society of Anesthesiologists. This is especially important since the AMA signed the Joint House-Senate Working Agreement that promised to develop about 140 performance measures covering about 34 clinical areas and, by 2007, that physicians would voluntarily report three to five measures each. Each of these measures has significant bearing on the practice of anesthesia as governmental pressures mount to pursue value-based purchasing (e.g., Deficit Reduction Act, Tax Relief Act) and potential financial incentives (Pay for Performance).

Mild hypothermia is often defined as about 34 to about 36 °C as measured at a core site (nasopharyx, esophageal, pulmonary artery, tympanic membrane thermocouple) and often will begin early in the preoperative period. Patients presenting for procedures are often anxious and then asked to disrobe, placed in a cool room wearing only a gown. Although many patients often complain of thermal discomfort during this preoperative time, there is little done other than providing passive warming with blankets. There have been studies that show significant improvement in the maintenance of normothermia by providing active warming in the preoperative period.

There are at least four types of heat loss pathways described as they relate to the perioperative period. These are evaporative, convective, radiant and conductive. All of these pathways work against normothermia in the perioperating arena. To combat these types of losses, humans have behavioral and autonomic responses to prevent hypothermia. When the patient enters the cold environment of the operating room he or she is unable to perform the normal behavioral response to thermal discomfort which consists of adding clothes or turning up the temperature in the room. On induction of anesthesia the autonomic function is inhibited.

Not to be forgotten is the thermal discomfort that many patients complain of during the perioperative process. As described above, this process often begins on admission for the procedure and can continue well into the recovery period. Although the warm blankets placed on the patient have an initial satisfaction, studies have shown the effect is brief. In summary, maintenance of perioperative normothermia has significant clinical importance as well as patient satisfaction issues, and may have an effect on payment for services for both the practitioner and the facility.

Because of the requirement for multiple machines, most facilities do not utilize the previously described forced air active warming device preoperatively and post operatively as a routine device. A forced air device is rarely found in the office based setting where about 25% of all elective procedures are now performed.

Currently the only active heating devices used in the perioperative arena are conventional forced air warmers.

Accordingly, there exists a need for warming blankets and methods of fabricating the same.

### SUMMARY OF THE INVENTION

Embodiments of the invention can address some or all of the above needs. Certain embodiments of the invention can provide warming blankets and methods of fabricating the same.

The invention is as set out in the claims.

Each of the respective warming elements may comprise carbon, charcoal, vermiculite, or an air-activated chemical composition.

In one aspect of the embodiment, the body has one of a substantially rectangular, a substantially square, a substantially elliptical, a substantially circular shape, or substantially conforms to the shape of a human body.

In one aspect of the embodiment, the body comprises one or more cut-out portions associated with one or more respective appendages of the human body.

A warming area provided by a first warming element may overlap with a warming area provided by a second warming element.

In one aspect of the embodiment, the body comprises at least two layers.

In one aspect of the embodiment, the blanket further comprises an insulating material situated between the at least two layers.

In one aspect of the embodiment, the body comprises a water resistant or waterproof material.

In one aspect of the embodiment, the body comprises a reflective material.

According to the invention each of the one or more pockets comprises an elastic or elasticized material.

In one aspect of the embodiment, each of the one or more pockets comprises a material that controls the flow of air to a heating element inserted into the pocket.

The one or more pockets may be arranged in a predetermined configuration such that overlapping regions of warmth are provided by the removable warming elements.

A method of using a warming blanket can include providing a warming blanket with one or more pockets adapted to hold a respective removable heating element. In addition, the method can include inserting a respective heating element into each of the one or more pockets. Furthermore, the method can include positioning the warming blanket on a user.

An easily opened, comfortable active warming device in the form of a sheet or blanket can be provided. The warming device can include a medical grade cloth that blends in with other operating room covers or drapes. It has a time course of at least 12 hours and is disposable once it cools. The warming device can be designed with a maximum temperature of 43°C and is actively warming within 20 minutes of opening. The warming device can include at least one application related to a medical procedure with an emphasis on providing pre, intra, and post operative warming for the clinical benefits as well as the patient satisfaction. The warming device can also find utility for non-medical use to prevent or otherwise minimize thermal discomfort in multiple settings.

A disposable medical grade cover or blanket having multiple independent energy sources produced via an exothermic chemical reaction that is initiated by exposure to air can be provided. The chemicals are safely contained in separate pockets (heating pods) in the cover that are strategically placed to heat the patient to provide both thermal comfort as well as a clinical warming effect. The blanket can provide active heat that is self contained in the blanket without requiring external attachments.

The blanket can provide a maximum temperature of 43°C and lasts for approximately 12 hours or more.

The cover can be a medical cloth, blue in color, and is 3 feet wide by 5 feet long.

The cover can have white stitching on its border.

The cover can come packaged in an airtight clear plastic 25.4cm (10 inch) by 43.18 cm (17 inch) package with the energy sources already in place.

The cover's packaging can be opened along a perforated easy open line, after which the blanket may be unfolded to its above size.

The cover can include energy sources configured so that the exothermic reaction takes place to provide noticeable warmth to the patient within about 20 minutes of opening.

The continuation of warming can occur until a plateau is reached after one hour. This exothermic reaction can continue for at least 12 hours.

The weight of the blanket can be such that the blanket will lie atop of the person without slipping off.

The cover can be fastened to the patient with at least one Velcro attachment at one end of the blanket. Other attachments can be utilized in other embodiments of the invention.

The cover can include three cutouts positioned to provide the ability to position the blanket either lengthwise (head to toe) on the patient or turning it 90° to allow exposure of abdomen and legs if the surgical procedure warrants this exposure.

The cover can include symmetrical positioning of the energy sources.

The cover can include asymmetrical positioning of the energy sources.

The cover can include symmetrical position of the energy sources across the width by asymmetrical positioning of the energy sources across the length.

Energy can be supplied via an exothermic chemical reaction that is initiated by exposure to air.

After activation of at least one heating element, the cover can be sealed in a relatively air tight enclosure to inhibit the exothermic reaction of the at least one heating element.

A method can be provided to facilitate patient comfort using an active warming device in the perioperative arena with emphasis on pre, intra, and post operative warming of the patient for multiple types of procedures that require general, neuraxial, local or sedation anesthesia.

Most facilities will utilize between 3 and 6 thick blankets per patient that presents for a procedure. This tremendous utilization of blankets results in large laundry bills for the facility that can eat into the profit margins. Certain embodiments of the invention can typically require only a single self-warming blanket. This could decrease the amount spent on laundry, as well as having an environmental effect by reducing water and energy utilization.

Other blankets and processes according to various embodiments of the invention will become apparent with respect to the remainder of this document.

### BRIEF DESCRIPTION OF THE DRAWINGS

Reference will now be made to the accompanying drawings, which are not necessarily drawn to scale, and wherein:
FIG. 1 is a schematic diagram of one example warming blanket or cover according to embodiment of the invention.
FIG. 2 is a schematic diagram of another example warming blanket or cover according to embodiment of the invention.
FIG. 3 is a schematic diagram of yet another example warming blanket or cover according to embodiment of the invention.
FIG. 4 is a schematic diagram of one example pocket for a warming blanket or cover that may be utilized to secure a heat pad according to an embodiment of the invention.
FIG. 5 is a flowchart of one example method for fabricating a warming blanket or cover according to an embodiment of the invention.
FIG. 6 is a flowchart of one example method for using a warming blanket or cover.
FIG. 7 is a top view of a folded self-warming blanket or cover packaged in an airtight polymer.
FIG. 8 is a top perspective view of the self-warming blanket or cover of FIG. 7 after removal from the packaging and unfolding.
FIG. 9 is a top plan view of another embodiment of a self-warming blanket or cover according to an embodiment of the invention.

### DETAILED DESCRIPTION OF EMBODIMENTS OF THE INVENTION

Embodiments of the invention now will be described more fully hereinafter with reference to the accompanying drawings. In the drawings, like numbers refer to like elements throughout.

Embodiments of the invention may provide improved warming blankets and methods of fabricating the warming blankets. Warming blankets in accordance with various embodiments of the invention include one or more pockets that facilitate the use of heat pads in association with the blankets. Each of the one or more pockets is configured to hold one or more heat pads. Each of the heat pads may be a heat pad that provides heat to at least a portion of a warming blanket or a heat zone of the warming blanket. In this regard, multiple heat pads may be utilized in association with a warming blanket, and the heat pads may be configured such that they have overlapping heat zones. Warming blankets in accordance with certain embodiments of the invention can provide increased warmth, drapability, conformability as well as comfort over certain conventional blankets in a variety of circumstances and applications.

Certain embodiments of the invention can eliminate or otherwise minimize the need to use a separate machine to provide thermal input for a patient. Such embodiments can be used to begin the pre-warming of a patient which may be shown to reduce the degree of cooling that the patient may experience during anesthesia. Furthermore, such embodiments can be used intraoperatively or as an adjunct to a forced air warmer and can provide significant thermal comfort and rewarming effect in the postoperative arena. In certain instances, such embodiments can be disposable.

As used herein, the terms "heating element", "warming element", "heat pad", "energy source", "heating pad", "warming pad", "pad", "pod", and their respective pluralized forms can be used interchangeably, and are intended to describe an air-activated carbon, charcoal, or vermiculite heat pocket. In some embodiments, a different air-activated material and/or chemical composition can be utilized.

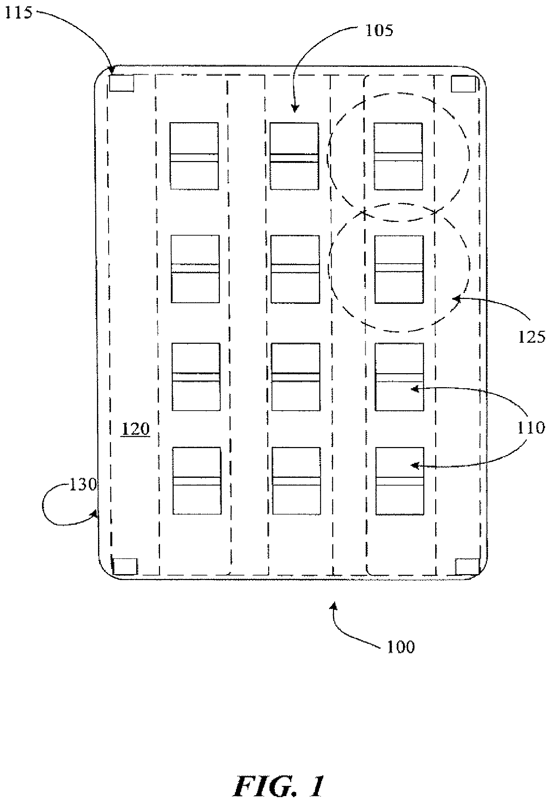

FIG. 1 is a schematic diagram of one example warming blanket 100 according to an embodiment of the invention. As shown in FIG. 1, the warming blanket 100 includes a body 105 and one or more pockets 110. Each of the one or more pockets 110 is attached to the body 105. As explained in greater detail below, each of the one or more pockets 110 is configured to hold one or more suitable heating elements or warming elements, such as one or more heat pads, heating pads, or warming pads.

A body 105 of a warming blanket 100 may have a wide variety of shapes and/or dimensions as desired in various embodiments of the invention. As shown in FIG. 1, the body 105 of the warming blanket 100 may have a substantially rectangular shape; however, in other embodiments of the invention, the body 105 may have a substantially square shape, substantially circular shape, substantially elliptical shape, a shape of a garment to be worn by a user or patient, or a shape that at least partially conforms to that of a user or patient, etc. Different shapes of warming blankets that are constructed from various materials may be designed to serve different functions. For example, a relatively large, substantially rectangular warming blanket may be constructed from relatively durable materials, and the warming blanket may be designed for outdoor and/or recreational uses. As another example, a relatively large, substantially rectangular blanket may be constructed from relatively lightweight, reflective materials such that the warming blanket can be easily packed or stored. In this regard, the warming blanket may be designed for use as an emergency blanket. As yet another example, a warming blanket may be formed with one or more cut-outs that conform to the shape of the human body, and the warming blanket may be designed for use in medical applications. In yet another embodiment, a relatively small size warming blanket with suitable cut-outs and/or material construction can be designed for use by an infant or child. In another embodiment, a warming blanket can be shaped to cover a person's torso in the form of a warming jacket. Other examples of warming blanket constructions and/or designs according to embodiments of the invention will be apparent.

According to an aspect of the invention, the body 105 includes a first surface 120 and a second surface 130. The first surface 120 of the body 105 may also be referred to as the internal surface of the warming blanket 100 or the back side of the warming blanket 100. Likewise, the back surface 130 of the body 105 may also be referred to as the external surface of the warming blanket 100 or the face side of the warming blanket 100. In certain embodiments of the invention, the second surface 130 may have a substantially smooth and/or have a substantially uniform surface. In use, the first surface 120 of the body 105 may be in direct contact with a user of the warming blanket 100. As shown in FIG. 1, one or more pockets 110 configured to hold respective heating or warming elements are attached or mounted to the first surface 120. In this regard, when the warming blanket 100 is in use, the heating or warming elements may provide additional heat to the user of the warming blanket 100. One suitable example of a heating or warming pad can be an air activated, carbon or charcoal powder filled pouch. In other embodiments, other types of individual heating or warming pads or air-activated materials or chemical compositions can be used.

In accordance with various embodiments of the invention, the warming blanket 100 may be constructed, manufactured, fabricated, and/or formed from a wide variety of different types of materials, different weights of materials, and/or combinations of different types and weights of materials. Additionally, as desired in various embodiments, the first surface 120 and the second surface 130 of the warming blanket 100 may be formed from different materials. For example, in one embodiment, the first surface 120 may be formed from one or more polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more breathable materials, one or more non-woven materials, one or more natural materials, one or more recycled or reworked materials, and/or one or more manmade or artificial materials. The second surface 130 may be formed from one or more polypropylene (PP) materials, spunbond (SB) materials, polypropylene spunbond (PPSB) materials, low density polyethylene (LDPE) materials, cotton, rayon, synthetics, one or more non-woven materials, one or more natural materials, and/or one or more manmade or artificial materials.

In one embodiment, certain weights of specific materials can be used for a warming blanket shown in FIG. 1. For example, a warming blanket can comprise a first surface of a white coated PPSB material with a weight of approximately 35 g/m2, and can further comprise a second surface of a dark blue LDPE material with a weight of approximately 30 g/m2. Various other embodiments can utilize different weights of similar or different materials depending at least in part on the desired comfort, drapability, conformability and/or warmth properties to be achieved.

Alternatively, the body 105 of the warming blanket 100 may be formed as a single component, and the first surface 120 and the second surface 130 may be formed utilizing the same or otherwise compatible materials and/or combinations of materials. Additionally, in certain embodiments, the first surface 120 and/or the second surface 130 may be constructed or formed to include a plurality of similar or different layers of material.

In any instance, the combination of materials selected for a warming blanket in accordance with certain embodiments of the invention can be based at least in part on the desired comfort, drapability, conformability and/or warmth for the warming blanket. For example, one or more predefined standards of comfort drapability, conformability and/or warmth may be accounted for in selecting the combination of materials for a warming blanket in accordance with an embodiment of the invention.

Furthermore, in other warming blanket embodiments, certain desired characteristics such as moisture permeability, oxygen transfer rates (OTR), and moisture vapor transmission rates (MVTR) can be accounted for and engineered in a warming blanket by selecting one or more suitable materials for assembly to achieve any desired combined characteristics for the assembled warming blanket.

According to one embodiment of the invention, the first surface 120 and/or the second surface 130 may be formed from one or more water resistant and/or waterproof materials. In another embodiment, the first surface 120 and/or the second surface 130 may include one or more layers of water resistant and/or waterproof material. In another embodiment, the first surface 120 and/or the second surface 130 may be coated with or otherwise treated with a water repellant.

In other embodiments of the invention, the first surface 120 and/or the second surface 130 may include a stain resistant layer and/or be treated with a stain resistant chemical or treatment. In yet other embodiments, the first surface 120 and/or the second surface 130 may include a reflective material or be treated to be reflective with a chemical, treatment, or layer. In this regard, the visibility of the blanket 100 may be enhanced.

In certain embodiments of the invention, the body 105 of the blanket 100 may include a single component with both a first surface 120 and a second surface 130. In other embodiments of the invention, the body 105 may include a plurality of components. For example, the first surface 120 and the second surface 130 of the body 105 may be separate components of the blanket 100. In embodiments where the body 105 is formed from a plurality of components, the components may be fixedly or removably connected and/or attached to one another via any suitable connectors, connecting techniques, and/or connection means. For example, the first surface 120 and the second surface 130 of the body 105 may be connected to one another via sewing, stitching, bonding, application of an adhesive, threads, ties, use of snaps, use of ultrasonic welding, use of velcro, use of one or more zippers, etc.

Additionally, in various embodiments of the invention, one or more insulation layers and/or insulating materials may be situated between the first surface 120 and the second surface 130. A wide variety of different insulating materials may be utilized as desired in various embodiments of the invention, such as, natural insulating materials (e.g., goose down), artificial insulating materials (e.g., Thermacore™ insulation) or reflective type materials (e.g. aluminum or tin reflective foil).

In accordance with one embodiment of the invention, the warming blanket 100 may include one or more weights 115 that may assist in holding the warming blanket 100 in place or with respect to a user or patient. For example, if the warming blanket is utilized in a medical or post-operative environment, the one or more weights 115 may assist in holding the warming blanket 100 down over a patient's body and minimizing the movement of the warming blanket 100 that is caused by movement of the patient or attending medical personnel. In another example, if the warming blanket is utilized in an outdoor environment (e.g., sporting event, picnic, etc.), the one or more weights 115 may assist in holding the warming blanket 100 down and minimizing the movement of the warming blanket 100 that is caused by external forces, such as, the wind. The one or more weights 115 may be situated at various locations on the warming blanket 100 as desired in various embodiments of the invention, such as, at one or more corners or along one or more edges of the warming blanket 100. As shown in FIG. 1, a weight 115 may be situated at each of the four corners of the warming blanket 100. Each of the one or more weights 115 may be permanently or removably affixed, attached, or otherwise mounted to the first surface 120 and/or the second surface 130 of the warming blanket by any suitable connectors, connecting techniques, and/or connection means. For example, each of the weights may be connected to the warming blanket 100 via sewing stitching, bonding, application of an adhesive, threads, ties, use of snaps, etc.

As shown in FIG. 1, one or more pockets 110 are attached to the first surface 120 of the warming blanket 100. Although the warming blanket 100 is described as having pockets 110 attached to the first surface 120 or internal surface of the body 105, various embodiments of the invention may include one or more pockets 110 that are attached to, mounted to, or affixed to the second surface 130 of the body 105. Additionally, in certain embodiments, one or more pockets 110 may be attached to the first surface 120 and/or the second surface 130 so that the one or more pockets 110 are situated between the first surface 120 and the second surface 130 (i.e., within the blanket). For example, in an embodiment where the first surface 120 and the second surface 130 are removably connected (e.g., via a zipper, etc.), the one or more pockets 110 are attached to the first surface 120 of the blanket, and between the first surface 120 and second surface 130 of the warming blanket 100.

One or more warming elements or heating elements may be situated in or placed in each of the one or more pockets 110. A wide variety of different warming or heating elements may be utilized as desired in various embodiments of the invention, such as, warming pads or heat pads. Additionally, a wide variety of different warming pads may be utilized as desired in various embodiments of the invention. Each of the warming elements may be an air activated charcoal and/or an air activated vermiculite warming pad. Each of the warming pads may be a disposable warming pad that contains a combination of materials such as iron powder, water, salt, activated charcoal, and/or vermiculite. When a warming pad is exposed to oxygen or otherwise activated, a chemical reaction may be initiated within the warming pad that causes heat to be emitted by the pad. The warming pad may continue to emit heat at a predetermined temperature, for example, about 120 to about 140 degrees Fahrenheit for a predetermined duration of time, for example, about 2 to about 30 hours. Furthermore, each warming pad may be configured so that an associated exothermic reaction takes place to provide noticeable warmth within about 20 minutes of activation.

Each of the one or more warming pads may be removably situated within or inserted into a pocket 110 of the warming blanket 100. When activated and situated within a pocket 110, a warming pad may emit heat that supplements any heat or warmth provided by or otherwise retained by the materials of the warming blanket 100. As shown in FIG. 1, each of the activated warming pads may provide heat or warmth to a certain area of the warming blanket 100. In other words, each of the activated warming pads may emit heat that affects the blanket 100 within a predefined heat zone 125 for the warming pad. Each of the predefined heat zones 125 may affect a substantially circular or other geometrically-shaped area of the warming blanket 100 that extends outwardly from the heat pad. Additionally, as shown in FIG. 1, various predefined heat zones 125 may overlap one another. In this regard, a plurality of warming pads may be utilized to provide heat or warmth to selected portions of a warming blanket or to all or substantially all of the warming blanket. In this manner, one or more overlapping predefined heat zones may provide additional heating or otherwise prolonged heating to certain or otherwise preselected areas of a user's body when the blanket 100 is worn by or placed over a user.

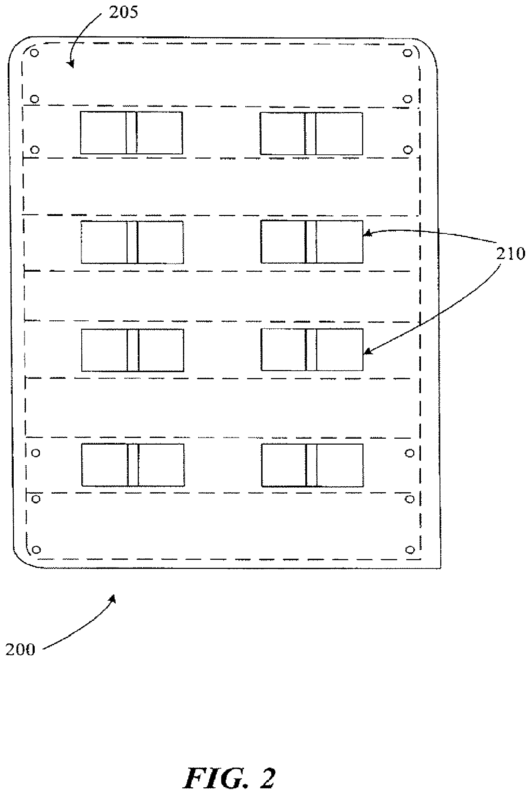

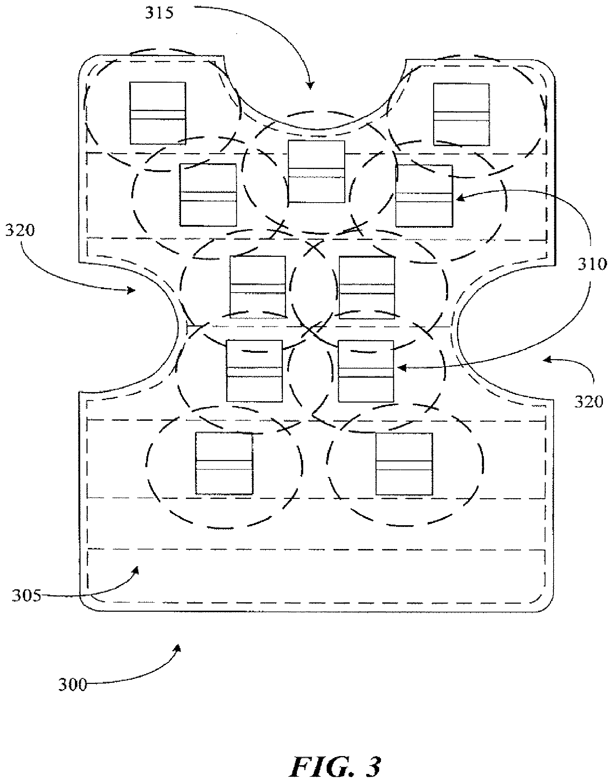

The one or more pockets 110 may be situated in a wide variety of different configurations as desired. In this regard, the predefined heat zones 125 emitted or generated by heat pads that are placed within the one or more pockets 110 may be configured in a wide variety of different ways as desired. A few examples of various configurations of the one or more pockets are illustrated in FIGS. 1-3. For example, in FIG. 1, four rows of three pockets each are utilized. As another example, in FIG. 2, four rows of two pockets each are utilized. As yet another example, in FIG. 3, eleven similar sized pockets may be situated in a specific geometric or non-geometric-shaped configuration to apply heat to predetermined areas of a user's body when heating or warming elements are mounted within the pockets and the blanket is worn by or placed over a user.

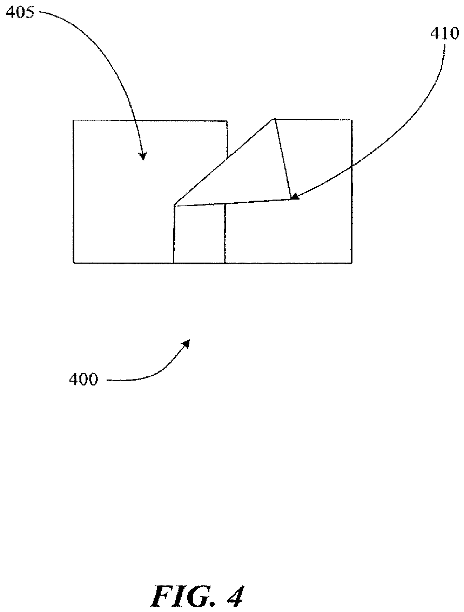

According to an aspect of the invention, each of the one or more pockets 110 is configured to hold at least one warming or heating element, such as a warming pad. According to certain embodiments of the invention, each of the one or more pockets 110 may have a design that is similar to that of a pillow sham, although other designs within the scope of the claim may be utilized as desired. The construction of one example pocket, such as pocket 110, is illustrated in FIG. 4 and described in the associated text below.

In certain embodiments of the invention, each of the one or more pockets 110 may have a shape that generally corresponds to the warming pads that are intended to be inserted into or situated with the pockets 110. As shown in FIG. 1, each of the one or more pockets may have a substantially rectangular shape; however, in other embodiments of the invention, each of the one or more pockets 110 may have a substantially square shape, a substantial circular shape, a substantially elliptical shape, etc. With continued reference to FIG. 1, each of the one or more substantially rectangular pockets 110 are situated such that the length dimension of the pockets 110 is substantially parallel to the length dimension of the warming blanket 100. However, in various embodiments of the invention, each of the pockets 110 may be situated as desired in a wide variety of different directions with respect to the blanket 100. For example, as shown in FIG. 2, each of the one or more pockets 110 may be situated such that the length dimension of the pockets 110 is substantially parallel to the width dimension of the warming blanket 100.

Each of the one or more pockets 110 may be constructed and/or formed from a wide variety of different materials or combinations of materials as desired in various embodiments of the invention, as long as the first and/or second portion include and/or are constructed at least in part from one or more elasticized or elastic materials. For example, each of the one or more pockets may be constructed from similar materials to those utilized to form or construct the first surface 120 of the warming blanket 100. In various embodiments of the invention, a pocket 110 may be formed from one or more polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more non-woven materials, one or more breathable materials, one or more natural materials, one or more recycled or reworked materials, and/or one or more manmade or artificial materials.

In one embodiment, certain weights of specific materials can be used for one or more pockets associated with a warming blanket shown in FIG. 1. For example, at least one pocket can comprise a nontreatment SMMS material with a weight of approximately 50 g/m2. Various other embodiments can utilize different weights of similar or different materials depending at least in part on the desired comfort, drapability, conformability and/or warmth properties to be achieved.

According to the invention, each of the one or more pockets 110 incorporates an elasticized or elastic material. The material may be stretched in order to place a warming pad within the pocket 110, and the material may then substantially return to its original position to hold the inserted heat pad in place. In this regard, an inserted warming pad may be prevented from shifting or moving within the pocket 110. Additionally, the elasticized material of the pocket 110 exerts a force on the warming pad that facilitates holding the warming pad and its contents in place. In this regard, the shifting of the internal ingredients of a warming pad (e.g., iron powder, salt, charcoal, etc.) may be reduced.

Additionally, in various embodiments of the invention, the one or more pockets 110 may be constructed from one or more materials that facilitate the control of airflow to warming pads inserted into the blanket 100. In this regard, the one or more pockets 110 may facilitate at least partial control over the duration of time in which inserted warming pads remain active. For example, by limiting and/or inhibiting the flow of air to a warming pad, the amount of oxygen provided to the warming pad may be limited, thereby facilitating a longer or shorter activation period for heat pad.

According to an aspect of the invention, one or more warming pads may be removably inserted into a pocket 110 and removed as needed. The one or more warming pads inserted into each respective pocket 110 may be replaced once their fuel source has been expended or the desired temperature from the respective pocket 110 needs to be modified. In this regard, the warming blanket 100 may be a reusable warming blanket 100 in which warming pads may be used and replaced prior to subsequent uses.

Each of the one or more pockets 110 is attached to the first surface 120 of the body 105 of the warming blanket 100 via any suitable connectors, connecting techniques, and/or connection means. For example, a pocket 110 may be connected to the body 105 of the warming blanket 100 via sewing, stitching, bonding, application of an adhesive, threads, ties, use of snaps, use of ultrasonic welding, use of velcro, use of one or more zippers, etc.

Certain weights of specific materials can be used for ties and/or threads associated with a warming blanket in FIG. 1. For example, at least one tie can comprise a white PPSB material with a weight of approximately 40 g/m2, and at least one thread can comprise a white spun 60/2 material. Different weights of similar or different materials depending at least in part on the desired comfort, drapability, conformability and/or warmth properties to be achieved can be utilised.

FIG. 2 is a schematic diagram of another example warming blanket 200 in accordance with an illustrative embodiment of the invention. The components of the warming blanket illustrated in FIG. 2 may be substantially similar to those of the warming blanket 100 illustrated in FIG. 1; however, the configuration of the pockets may be different. As shown in FIG. 2, the warming blanket 200 may have a body 205 with a plurality of pockets 210 connected to the body 205. The warming blanket 200 of FIG. 2 includes a fewer number of pockets 210 than those illustrated for the warming blanket 100 of in FIG. 1. The use of a fewer number of pockets 210, along with fewer materials used to construct the warming blanket 200, may lead to a warming blanket 200 that has a relatively lower overall weight. If a fewer number of warming pads are inserted into a warming blanket 200, then a warming blanket in use will have a relatively lower weight. Additionally, the use of a fewer number of warming pads may lessen the warmth or heat provided by the warming blanket 200. A warming blanket can include a particular number of warming pads configured so that the associated exothermic reaction with each respective pad takes place to provide noticeable warmth within about 10 to 20 minutes of activation. Accordingly, during the design of a warming blanket, the size of the warming blanket, desired heat region to be provided by the warming pads, and/or the weight of the warming blanket may be taken into account.

Certain weights of specific materials can be used for a warming blanket shown in FIG. 2. For example, a warming blanket can comprise a first surface of a PPSB material with a weight of approximately 40 g/m2, and can further comprise a second surface of a coated blue #Y542U LDPE material with a weight of approximately 20 g/m2. Different weights of similar or different materials depending at least in part on the comfort, drapability, conformability, and/or warmth properties to be achieved can be utilised.

Certain weights of specific materials can be used for one or more pockets associated with a warming blanket shown in FIG. 2. For example, at least one pocket can comprise a nontreatment white #15 SMMS material with a weight of approximately 50 g/m2. Different weights of similar or different materials depending at least in part on the comfort, drapability, conformability and/or warmth properties to be achieved can be utilised.

Certain weights of specific materials can be used for ties and/or threads associated with a warming blanket in FIG. 2. For example, at least one tie can comprise a white PPSB material with a weight of approximately 38 g/m2, and at least one thread can comprise a white spun 60/2 material. Different weights of similar or different materials depending at least in part on the comfort, drapability, conformability and/or warmth properties to be achieved can be utilised.

With continued reference to FIG. 2, the warming blanket 200 illustrated may be utilized, for example, as an emergency blanket. The incorporation of fewer pockets 210 may lead to a less bulky and relatively lower weight blanket that may be packed or stowed in an emergency kit, hiking pack, vehicle, etc. Additionally, the warming blanket 200 may be constructed from relatively light weight reflective materials, as described above.

Additionally, the warming blanket 200 may include one or more components that facilitate the folding, packing, and/or storage of the warming blanket 200. For example, one or more snaps, zippers, buttons, etc. may be positioned on the warming blanket 200 to facilitate maintaining the warming blanket 200 in a folded and/or packable configuration. The warming blanket 200 may include one or more snaps situated at one or more corners of the warming blanket 200, and the one or more snaps may be utilized to maintain the warming blanket 200 in a folded position. The warming blanket 200 may include a storage portion or pocket portion, and the warming blanket 200 may be inserted into or packed into the storage portion. In this regard, the warming blanket 200 may be packed and/or stored in a relatively portable configuration.

FIG. 3 is a schematic diagram of yet another example warming blanket 300 in accordance with an illustrative embodiment of the invention. The components of the warming blanket illustrated in FIG. 3 may be substantially similar to those of the warming blanket 100 illustrated in FIG. 1. However, as with FIG. 2, the configuration of the pockets may be different. As shown in FIG. 3, the warming blanket 300 includes a body 305 with a plurality of pockets 310 connected to the first surface of the body 305. The plurality of pockets 310 may be arranged in a configuration that provides a desired warming area or a desired group of heat zones for the warming blanket 300.

The plurality of pockets 310 may be configured to provide warmth, or heat to a patient in association with various medical and/or surgical procedures, such as, pre-operative, intra-operative and/or post-operative procedures. The plurality of pockets 310 may be situated in such a manner that relatively optimal heating or warming is provided to a patient. Additionally, the plurality of pockets 310 may be situated such that overlapping regions or areas of warmth are provided by associated warming pads. The configuration and/or locations of the pockets 310 and their associated overlapping regions of warmth may be predetermined in order to provide relatively optimal heating to a patient. Furthermore, a warming blanket can include a certain number of warming pads configured so that the associated exothermic reaction with each respective pad takes place to provide noticeable warmth within about 10 to 20 minutes of activation.

Certain weights of specific materials can be used for one or more pockets associated with a warming blanket shown in FIG. 3. For example, at least one pocket can comprise a nontreatment blue #15 SMMS material with a weight of approximately 52 g/m2. Different weights of similar or different materials depending at least in part on the comfort, drapability, conformability and/or warmth properties to be achieved can be utilised.

Certain weights of specific materials can be used for ties and/or threads associated with a warming blanket in FIG. 3. For example, at least one tie can comprise a white PPSB material with a weight of approximately 40 g/m2, and at least one thread can comprise a white spun 60/2 material and/or a spun Venus white material. Different weights of similar or different materials depending at least in part on the comfort, drapability, conformability and/or warmth properties to be achieved can be utilised.

Additionally, the body 305 of the warming blanket 300 may include one or more cut-out portions 315, 320 that are associated with the head and/or limbs of a patient or user of the warming blanket 300. As shown in FIG. 3, a cut-out 315 may be provided for a patient's head and two respective cut-outs 320 may be provided for a patient's arms. By providing these cut-outs, the warming blanket 300 will facilitate accommodating protruding portions of a patient's body. In this regard, a desired warming of certain portions of a patient's body may be achieved in a medical-type or other application. Additionally, the cut-outs can facilitate greater flexibility in die movement of the head and/or limbs of the patient while maintaining the application of heat to certain other portions of a patient's body.

Warming blankets in accordance with various embodiments of the invention may be utilized in a wide variety of different applications and/or for a wide variety of different purposes. Some examples of applications in which the warming blankets may be utilized are outdoor activities, recreational activities, sporting activities, travel activities, emergency situations, and or medical applications. Additionally, the warming blankets may be utilized to provide a wide variety of different benefits to a user, such as, comfort, warmth, protection from the elements, stress relief, muscle tension relief, therapeutic purposes, aid in circulation, arthritis relief, etc.

As discussed above, the combination of materials selected for a warming blanket in accordance with certain embodiments of the invention can be based at least in part on comfort drapability, conformability and/or warmth. For example, one or more predefined standards of comfort, drapability, conformability and/or warmth may be accounted for in selecting the combination of materials for a warming blanket in accordance with an embodiment of the invention.

Furthermore, in other warming blanket embodiments, certain characteristics such as moisture permeability, oxygen transfer rates (OTR), and moisture vapor transmission rates (MVTR) can be accounted for and engineered in a warming blanket by selecting one or more suitable materials for assembly to achieve any desired combined characteristic for the assembled warming blanket.

FIG. 4 is a schematic diagram of one example pocket 400 that may be utilized to secure a warming pad in accordance with various embodiments of the invention. As shown in FIG. 4, the pocket 400 includes a first portion 405 and a second portion 410. The first portion 405 extends from one edge of the pocket 400 and the second portion 410 extends from an opposite edge of the pocket 400. The first portion 405 and the second portion 410 may overlap one another. The amount of overlap may vary as desired. For example, the amount of overlap may be approximately 2.0 cm (or less than about one inch). Alternately, there may be no overlap between the first portion 405 and the second portion 410.

By providing a first portion 405 and a second portion 410 of a pocket 400, the pocket 400 facilitates the insertion of a warming pad, and the pocket maintains or holds an inserted warming pad in place. A warming pad may be inserted between the first portion 405 and the second portion 410 of the pocket 400, and the first portion 405 and/or the second portion 410 are adjustable or otherwise manipulable such that the warming pad is situated beneath both the first portion 405 and the second portion 410 of the pocket 400. In this regard, a warming pad may be removably secured within the pocket 400.

According to the invention, the first portion 405 and/or the second portion 410 include and/or are constructed at least in part from one or more elasticized or elastic materials. In this regard, the first portion 405 and/or the second portion 410 is flexible, thereby permitting the insertion of one or more warming pads. Additionally, the first portion 405 and/or the second portion 410 supplies a compressive force to an inserted heat pad that facilitates holding the warming pad and/or its internal components in place.

In addition to the first portion 405 and the second portion 410 of the pocket 400, which may form an outer surface of the pocket 400, the pocket 400 may include an internal surface (not shown). The internal surface may be configured to contact the body of a warming blanket in which the pocket 400 is utilized. Additionally, the internal surface may provide additional support to a warming pad or other warming element that is inserted into the pocket 400.

The pocket facilitates a relatively easy insertion and removal of a warming pad into and out of the pocket. Additionally, the pocket facilitates the retention of the warming pad in place once the warming pad is inserted.

Additionally, a pocket may be formed or constructed from one or more materials that are suitable for medical applications, such as, polypropylene (PP) materials, spunbond (SB) materials, spunbond/melt blown/spunbond (SMS) materials, spunbond/melt blown/melt blown/spunbond (SMMS) materials, low density polyethylene (LDPE) materials, cotton, rayon, synthetics, one or more non-woven materials, one or more breathable materials, one or more natural materials, one or more recycled or reworked materials, and/or one or more manmade or artificial materials, as long as the first and/or second portion includes and/or is constructed at least in part from one or more elasticized or elastic materials. The materials utilized in the construction of the pocket may be relatively sterile materials that facilitate cleanliness within a medical environment.

Additionally, a pocket may be formed without fastenings, such as zippers. For example, a pocket that is incorporated into a warming blanket utilized for various medical applications may be formed without fastenings. The absence of fastenings may minimize other objects snagging or catching the blanket, and may further facilitate the ease and flexibility of inserting and removing warmers from the pockets. Additionally, the absence of fastenings may provide a pocket with a relatively higher sterility and/or cleanliness as compared to a pocket that includes one or more fastenings.

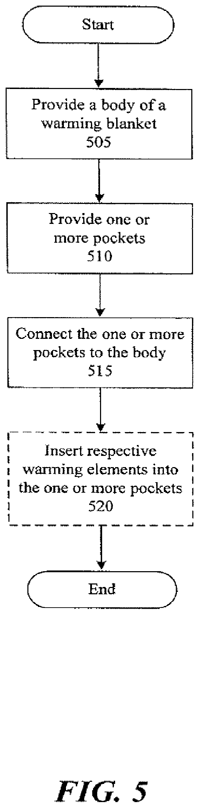

FIG. 5 is a flowchart of one example method 500 for fabricating a warming blanket in accordance with various embodiments of the invention. The method 500 may begin at block 505.

At block 505, a body of a warming blanket is provided. The body may be formed from a wide variety of different materials and/or combinations of materials as desired in various embodiments of the invention, as discussed in greater detail above with reference to FIGs. 1 - 3.

At block 510, one or more pockets are provided in accordance with claim 1.

At block 515, the one or more pockets are mounted to the body via any number of suitable connections, connection techniques, and/or connection means, as discussed in greater detail above with reference to FIGs. 1 - 4. The one or more pockets may be mounted to the body in a wide variety of different configurations that may determine at least in part the heating area that is provided by the warming blanket once one or more heat pads are inserted.

Once the one or more pockets have been mounted to the body at block 515, operations may continue at block 520, which is optional. At block 520, respective warming elements, such as heat pads, may be inserted into one or more of the pockets that have been mounted to the body of the blanket.

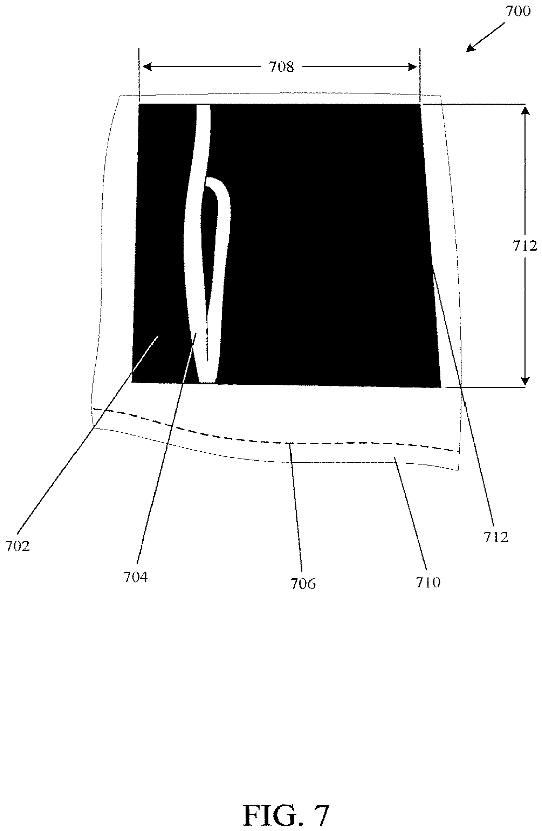

Once constructed, a warming blanket may be situated in a suitable package or within a suitable packaging material for distribution. For example, a constructed blanket may be placed in an air-tight sealed package for distribution as a ready-to-use blanket, an example of which is described and shown in FIG. 7 below.

The method 500 may end following block 520.

The operations described in the method 500 of FIG. 5 do not necessarily have to be performed in the order set forth in FIG. 5, but instead may be performed in any suitable order.

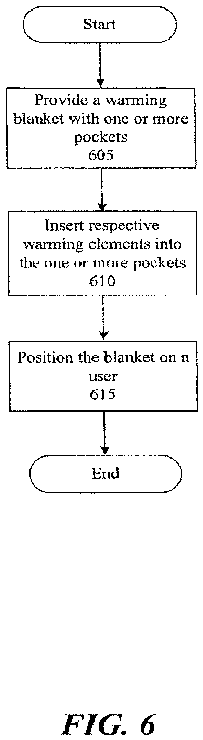

FIG. 6 is a flowchart of one example method 600 for using a warming blanket. The method 600 may begin at block 605.

At block 605, a warming blanket may be provided. The warming blanket may include one or more pockets that are configured or adapted to have a respective warming element, such as, a warming pad, inserted to rest within the pocket. Additionally, the pockets may be arranged in a predetermined configuration on the warming blanket to provide predefined heat zones or overlapping predefined heat zones. In some embodiments, the warming blanket may also include one or more cut-out portions as described above with reference to FIG. 3.

At block 610, respective warming elements, such as warming pads, may be inserted into one or more of the pockets of the provided warming blanket. Each of the respective warming elements may be activated either before or after they are inserted into a pocket.

The cover can be prepackaged wherein the cover comprises a plurality of pouches that each include a self-warming chemical composition when exposed to air. When the packaging is opened, the cover can be exposed to air, and the plurality of pouches can be activated to generate heat.

At block 615, the warming blanket may be positioned on one or more users of the blanket. For example, in a medical application, the warming blanket may be positioned with respect to or mounted directly on or with respect to a portion of a user or patient's body. In this manner, the warming blanket can provide predefined heat zones or overlapping predefined heat zones to a user or patient's body.

The method 600 can include providing an anesthetic to the user or patient.

The cover can be positioned adjacent to the patient in a pre-operative room.

The cover can be positioned adjacent to the patient in a post-operative room.

The cover can be exposed to air approximately 20 minutes before positioning the cover adjacent to the patient.

The cover can be self-warmed by an exothermic reaction in the chemical composition in each pouch, and wherein the exothermic reaction lasts approximately 12 hours.

The cover can be positioned in a first orientation in a pre-operative room, and is shifted to a second orientation in an intraoperative room.

After activation of one or more warming elements, such as warming pads, the cover can be sealed in a relatively air tight enclosure to inhibit or otherwise cease the exothermic reaction associated with the warming elements.

The method 600 may end following block 615.

The operations described in the method 600 of FIG. 6 do not necessarily have to be performed in the order set forth in FIG. 6, but instead may be performed in any suitable order. Additionally, more or less than all of the operations set forth in FIG. 6 may be performed.

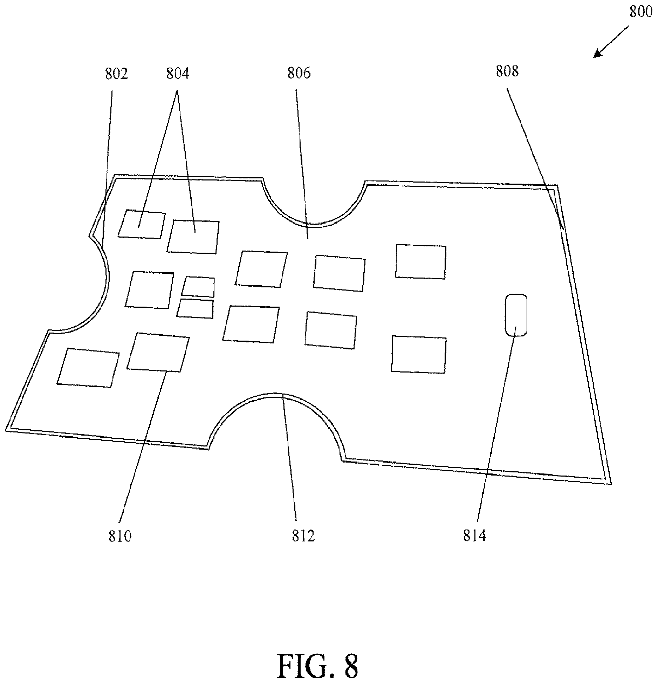

With reference to FIGs. 7 and 8 there is illustrated a blanket or cover in a folded and packaged format, shown as 700 in FIG. 7, and an unfolded cover 800 shown in FIG. 8 removed from the packaging. As shown in FIGs. 7 and 8, a cover 702, 800 can have medical grade consistency and color, such as blue. Outer white stitching can be in keeping with normal drapes and covers seen in typical operating rooms and other medical facilities. The size of the unfolded cover 800 shown in FIG. 8 can be about 3 feet by about 5 feet.

With reference to FIG. 7 there is illustrated a self-warming cover 702 enclosed within relatively airtight packaging 710. Packaging 710 can be relatively clear plastic, or any relatively impermeable durable material. The self-warming cover 702 can include an attachment portion 704 placed at a head end of the cover 702. The attachment portion 704 can be a loop intended to be draped around the head of a user or patient. The loop may be made of the same material as the cover's main body, or of the material used to form the border of the cover. Materials used in manufacturing the blanket described in FIGs. 1 - 4 above can be used for the cover 702, 800 described herein. The loop might be permanently attached to the border of the main body of the cover 702. Alternatively, one or both ends of the attachment portion 704 might be removably attached to the main body of the cover 702 or its border, for example via a velcro fastener. The attachment portion 704 might be a relatively simple tie string that is knotted. Two strings permanently attached to a cover, such as 702, might be used and then tied together or attached at respective adhesive ends. A separate cord with clips or other mounting devices at the ends can attach to the main body of the cover, such as 702.

The cover 702 shown in FIG. 7 can be oriented within associated packaging 710 so that upon opening of package 710 the attachment portion 704 is readily accessible. Packaging 710 can include an area 706, such as a perforated line, for relatively easy opening. However, it should be understood that instead of perforations or scoring, the packaging 710 might include a weakened or thinned area or line. The packaging 710 might forego such an area 706, and scissors or a knife may be needed in order to open the packaging 710. The packaging 710 may include a sealing device, such as a zip lock mechanism, which may facilitate resealing the packaging 710 to exclude or otherwise inhibit exposure of the cover, such 702, therein from outside air. Packaging 710 is illustrated as substantially clear, but opaque packaging might also be used. Vacuum or other forms of packaging potentially can significantly decrease storage space utilization at hospitals and other healthcare providers. Furthermore, it should be understood that each package can be bar coded, tagged by a RFID tag or other tracking technology, or otherwise marked for more accurate tracking, utilization and billing purposes.

As previously noted, packaging 710 can enclose the cover 702 in a relatively airtight environment to prevent or otherwise minimize premature activation of the exothermic chemical reaction that can occur in energy sources placed within various pockets or pouches of the cover 702. A cover can include a certain number of energy sources, such as warming pads, configured so that the associated exothermic reaction with each respective source or pad takes place to provide noticeable warmth within about 10 to 20 minutes of activation. After activation of one or more energy sources, such as warming pads, the cover can be sealed in a relatively air tight enclosure to inhibit or otherwise cease the exothermic reaction associated with the energy sources, and the cover can be reused with the energy sources at a later time upon exposure to air. The width of a folded cover 702 within the packaging 710 is designated by reference numeral 708, and is approximately 25.4cm (ten inches). The length of a folded cover 702 within the packaging 710 is designated by reference numeral 712, and is approximately 43.18cm (seventeen inches).

With reference to FIG. 8, there is illustrated an unfolded cover 800. The cover 800 can be colored blue and with medical grade cloth consistency. The cover 800 has a superior or upper portion 802 with a cut out for a patient's head. An attachment portion, similar to the attachment portion 704 previously described in FIG. 7, can be placed just lateral to the edge of cutout to allow the user to secure the cover 800 and to prevent it from slipping down from a user or patient's neck. The cover 800 can include a plurality of upper heating pads 804 strategically placed to provide warmth to the chest and upper body of a user or patient beneath the cover 800. The cover 800 further can include one or more heating pods placed in pockets in the central or abdominal region as well as lower portions of the cover 800. Opposing end 808 of the cover 800 is opposite the superior or upper portion 802 of the cover 800.

As should be seen in FIG. 8, cover 800 can be asymmetrical with respect to the heating pads. That is to say, the opposing end 808 may not include as many heating pods (if any) as the superior or upper portion 802 of the cover 800. This can provide the ability to flip the inferior portion 814 of cover 800 upward when the cover is in about 90 degree axis mode to allow access to extremity. As illustrated in FIG. 8, the cover 800 can include two cutouts 806, 812 on each of the sides of the cover 800 extending between the lower end 808 and the opposing or upper portion 802 of the cover 800. The cutouts 806, 812 are intended for about 90 degree axis mode, allowing the cover 800 to function intraoperatively for abdominal, lower extremity and upper extremity surgical procedures. Cutouts 806, 812 still allow for complete coverage of average size patient when utilized in long axis mode.

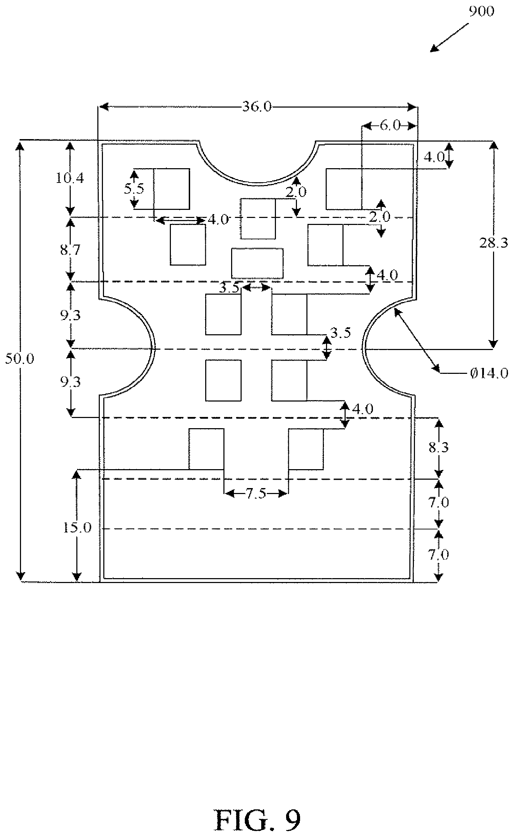

With reference to FIG. 9, there is illustrated various aspects of the dimensions and distributions of pouches and heating pads for an example cover 900.

It should be understood that variations in the size (length and width), weight and color of the cover 900 can be possible. The cutouts might take a wide variety of sizes and shapes, however the size and shape as illustrated in FIGs. 8 and 9 are suitable in certain instances for access to the patient if the covers 800, 900 are also used during the intraoperative time period.

Similarly, the placement of the heating pads could be changed. The heating pods can be positioned within pockets or pouches of the cover 900. Such heating pods might be relatively flat packets having some minimal thickness, but can take on other configurations. Heating pads might be positioned within the cover 900 during manufacture of the cover 900, or might be added in to pouches or pockets that are fastened, sewed or welded shut after placement of the heating pads. Heating pads suitable for use with the covers 800, 900 shown in FIGs. 8 and 9 are described above with respect to FIGs. 1 - 4. The heating pads or warmers can be those manufactured by or otherwise distributed by MyCoal Corporation of Japan, and the exothermic chemical reaction of the same is induced by exposure to air.

Warmers, such as warming pads, can be configured so that the associated exothermic reaction with each respective pad takes place to provide noticeable warmth within any predetermined amount of time after activation, for example, within about 10 to 20 minutes. Such warmers or warming pads might be selected so as to lengthen or shorten the duration of exothermic reaction from the about 12 hour duration discussed herein. For example, for short elective procedures a shorter time duration might be suitable. However, it should be understood that such duration can be selected with the intent that a user or patient might want to take the cover 900 or blanket home and make use of the same for hours after departing the location of the procedure.

It should be understood that the shape of the example covers 800, 900 or blankets as illustrated in FIGs. 8 and 9 are specifically suited for the perioperative environment. Similarly, the placement of heat pads illustrated therein provides for heat distribution that will provide the suitable thermal comfort yet simultaneously allow for surgical access to a user or patient. Other shapes and sizes are contemplated as within the scope of the claimed invention. For example, it is contemplated as within the scope of the claimed invention that a cover tailored solely to overlap the lower extremities might be manufactured for use in surgery or other procedures in which access to the entire torso is desired. Similarly, it is contemplated as within the scope of the claimed invention that a cover tailored solely to overlap the torso might be manufactured for use in surgery or other procedures in which access only to the lower extremities is desired.

Furthermore, it is contemplated that the chemical composition of the warming pads could be spread out to encompass the entire cover to provide all areas of the blanket with an exothermic reaction. An increase in production costs, as well as weight, may result.

The cover 800, 900 may be laid upon. It is contemplated that its width might be made greater and that the absence of heating pods on the extended width might permit such width to be positioned beneath the patient if desired.

It should be understood that the cover 800, 900 can be, but may not necessarily, be disposable. In one form, the cover or sheet would be biodegradable to reduce the amount of paper or other waste (the cover is not necessarily a paper product and may be manufactured from a polymer or other material).

The methods 500, 600 shown in FIGs. 5 and 6 for fabricating and using blankets and covers can be applied to the covers 800, 900 shown in FIGs. 8 and 9.

## Claims

1. A warming blanket (100, 200, 300, 700, 800, 900), comprising:
a body (105, 205, 305) comprising a first surface (120); and
one or more pockets (110, 210, 310, 400) attached to the first surface, wherein each of the one or more pockets is adapted to hold a respective removable warming element;
**characterized in that** one or more of the pockets are adapted so that the said heating element is removably insertable into the pocket, wherein the pocket comprises a first portion (405) extending from one edge of the pocket and a second portion (410) extending from the opposite edge wherein the heating element is insertable between the first portion and the second portion, and the first portion and/or the second portion are adjustable or manipulable to situate the heating element beneath both the first portion and the second portion, wherein the first portion and/or the second portion include and/or are constructed at least in part from one or more elasticized or elastic materials and the first portion and/or the second portion are flexible, thereby permitting the insertion of the heating element and the first portion and/or the second portion supply a compressive force to the warming element.

2. The warming blanket (100, 200, 300, 700, 800, 900) of claim 1, wherein the body (105, 205, 305) has one of a substantially rectangular, a substantially square, a substantially elliptical, a substantially circular shape, a garment shape, or a shape that substantially conforms to the shape of a human body.

3. The warming blanket (100, 200, 300, 700, 800, 900) of claim 2, wherein the body (105, 205, 305) comprises one or more cut-out portions to accommodate one or more respective appendages of the human body.

4. The warming blanket (100, 200, 300, 700, 800, 900) of claim 1, wherein the body (105, 205, 305) comprises at least two layers.

5. The warming blanket (100, 200, 300, 700, 800, 900) of claim 4, wherein the blanket further comprises an insulating material disposed between the at least two layers.

6. The warming blanket (100, 200, 300, 700, 800, 900) of claim 1, wherein the body (105, 205, 305) comprises a water resistant or waterproof material.

7. The warming blanket (100, 200, 300, 700, 800, 900) of claim 1, wherein the body (105, 205, 305) comprises at least one reflective material.

8. The warming blanket (100, 200, 300, 700, 800, 900) of claim 1, wherein each of the one or more pockets (110, 210, 310, 400) comprises a material that controls the flow of air to a heating element inserted into the pocket.

9. A method for fabricating a warming blanket (100, 200, 300, 700, 800, 900) according to claim 1, comprising:
providing a body (105, 205, 305) comprising a first surface (120) for the warming blanket;
providing one or more pockets (110, 210, 310, 400), wherein each of the one or more pockets is adapted to hold a respective removable heating element; and
mounting the one or more pockets to the body;
**characterized in that** one or more of the pockets are adapted so that the said heating element is removably insertable into the pocket, wherein the pocket comprises a first portion (405) extending from one edge of the pocket and a second portion (410) extending from the opposite edge wherein the heating element is insertable between the first portion and the second portion and the first portion and/or the second portion may be adjusted or manipulated such that the heating element is situated beneath both the first portion and the second portion and the first portion and/or the second portion supplies a compressive force to the heating element.

## Patentansprüche

1. Wärmedecke (100, 200, 300, 700, 800, 900), Folgendes umfassend:
einen Körper (105, 205, 305), eine erste Oberfläche umfassend (120); und
ein oder mehrere Fächer (110, 210, 310, 400), die an der ersten Oberfläche angebracht sind, wobei jedes des einen oder der mehreren Fächer angepasst ist, ein jeweiliges entfernbares Wärmeelement zu fassen;
**dadurch gekennzeichnet, dass** eines oder mehrere der Fächer derart angepasst sind, dass das Wärmeelement entfernbar in das Fach einsetzbar ist, wobei das Fach einen ersten Abschnitt (405), der sich von einer Kante des Faches erstreckt, und einen zweiten Abschnitt (410) umfasst, der sich von der gegenüberliegenden Kante erstreckt, wobei das Wärmeelement zwischen dem ersten Abschnitt und dem zweiten Abschnitt einsetzbar ist, und der erste Abschnitt und/oder der zweite Abschnitt anpassbar oder manipulierbar ist, um das Wärmeelement sowohl unter dem ersten Abschnitt als auch unter dem zweiten Abschnitt unterzubringen, wobei der erste Abschnitt und/oder der zweite Abschnitt eines oder mehrere elastische oder nachgiebige Materialien enthält und/oder aus diesen hergestellt ist und der erste Abschnitt und/oder der zweite Abschnitt flexibel ist, und demnach das Einsetzen des Wärmeelementes erlaubt, und wobei der erste Abschnitt und/oder der zweite Abschnitt das Wärmeelement mit einer Druckkraft versieht.

2. Wärmedecke (100, 200, 300, 700, 800, 900) nach Anspruch 1, wobei der Körper (105, 205, 305) eine der Folgenden aufweist: eine im Wesentlichen rechteckige, eine im Wesentlichen quadratische, eine im Wesentlichen elliptische, eine im Wesentlichen kreisförmige Form, eine Form eines Kleidungsstückes oder eine Form, die im Wesentlichen mit der Form eines menschlichen Körpers übereinstimmt.

3. Wärmedecke (100, 200, 300, 700, 800, 900) nach Anspruch 2, wobei der Körper (105, 205, 305) einen oder mehrere ausgeschnittene Abschnitte umfasst, um ein oder mehrere Gliedmaßen des menschlichen Körpers aufzunehmen.

4. Wärmedecke (100, 200, 300, 700, 800, 900) nach Anspruch 1, wobei der Körper (105, 205, 305) wenigstens zwei Schichten umfasst.

5. Wärmedecke (100, 200, 300, 700, 800, 900) nach Anspruch 4, wobei die Decke ferner ein isolierendes Material umfasst, das zwischen den wenigstens zwei Schichten angeordnet ist.

6. Wärmedecke (100, 200, 300, 700, 800, 900) nach Anspruch 1, wobei der Körper (105, 205, 305) ein wasserresistentes oder wasserdichtes Material umfasst.

7. Wärmedecke (100, 200, 300, 700, 800, 900) nach Anspruch 1, wobei der Körper (105, 205, 305) wenigstens ein reflektierendes Material umfasst.

8. Wärmedecke (100, 200, 300, 700, 800, 900) nach Anspruch 1, wobei jedes des einen oder der mehreren Fächer (110, 210, 310, 400) ein Material umfasst, das den Luftstrom zu einem in das Fach eingesetzten Wärmeelement steuert.

9. Verfahren zum Herstellen einer Wärmedecke (100, 200, 300, 700, 800, 900) nach Anspruch 1, Folgendes umfassend:
Bereitstellen eines Körpers (105, 205, 305), eine erste Oberfläche (120) für die Wärmedecke umfassend;
Bereitstellen eines oder mehrerer Fächer (110, 210, 310, 400), wobei jedes des einen oder der mehreren Fächer angepasst ist, ein jeweiliges entfernbares Wärmeelement zu fassen; und
Lagern des einen oder der mehreren Fächer an dem Körper;
**dadurch gekennzeichnet, dass** eines oder mehrere der Fächer derart angepasst sind, dass das besagte Wärmeelement entfernbar in das Fach einsetzbar ist, wobei das Fach einen ersten Abschnitt (405), der sich von einer Kante des Faches erstreckt, und einen zweiten Abschnitt (410) umfasst, der sich von der gegenüberliegenden Kante erstreckt, wobei das Wärmeelement zwischen dem ersten Abschnitt und dem zweiten Abschnitt einsetzbar ist, und der erste Abschnitt und/oder der zweite Abschnitt derart angepasst oder manipuliert werden kann, dass das Wärmeelement sowohl unter dem ersten Abschnitt als auch unter dem zweiten Abschnitt untergebracht ist, und wobei der erste Abschnitt und/oder der zweite Abschnitt das Wärmeelement mit einer Druckkraft versieht.

## Revendications

1. Couverture chauffante (100, 200, 300, 700, 800, 900), comprenant :
un corps (105, 205, 305) comprenant une première surface (120) ; et
une ou plusieurs poches (110, 210, 310, 400) attachées à la première surface, chaque poche parmi la ou les poches étant conçue pour contenir un élément chauffant amovible respectif ;
**caractérisée en ce que** la ou les poches sont conçues de telle sorte que ledit élément chauffant soit insérable de manière amovible dans la poche, la poche comprenant une première partie (405) qui s'étend depuis un bord de la poche et une seconde partie (410) qui s'étend depuis le bord opposé, l'élément de chauffage étant insérable entre la première partie et la seconde partie, et la première partie et/ou la seconde partie étant ajustables ou manipulables pour situer l'élément chauffant sous la première partie, et la seconde partie, la première partie et/ou la seconde partie comprenant et/ou étant constitués au moins en partie d'un ou de plusieurs matériaux élastifiés ou élastiques, et la première partie et/ou la seconde partie étant flexibles, permettant ainsi l'insertion de l'élément chauffant, et la première partie et/ou la seconde partie appliquant une force de compression sur l'élément chauffant.

2. Couverture chauffante (100, 200, 300, 700, 800, 900) selon la revendication 1, dans laquelle le corps (105, 205, 305) a une forme parmi une forme sensiblement rectangulaire, une forme sensiblement carrée, une forme sensiblement elliptique, une forme sensiblement circulaire, une forme de vêtement ou une forme sensiblement conforme à la forme d'un corps humain.

3. Couverture chauffante (100, 200, 300, 700, 800, 900) selon la revendication 2, dans laquelle le corps (105, 205, 305) comprend une ou plusieurs parties découpées pour recevoir un ou plusieurs appendices du corps humain.

4. Couverture chauffante (100, 200, 300, 700, 800, 900) selon la revendication 1, dans laquelle le corps (105, 205, 305) comprend au moins deux couches.

5. Couverture chauffante (100, 200, 300, 700, 800, 900) selon la revendication 4, la couverture comprenant en outre un matériau isolant disposé entre les au moins deux couches.

6. Couverture chauffante (100, 200, 300, 700, 800, 900) selon la revendication 1, dans laquelle le corps (105, 205, 305) comprend un matériau résistant à l'eau ou imperméable.

7. Couverture chauffante (100, 200, 300, 700, 800, 900) selon la revendication 1, dans laquelle le corps (105, 205, 305) comprend au moins un matériau réfléchissant.

8. Couverture chauffante (100, 200, 300, 700, 800, 900) selon la revendication 1, dans laquelle chaque poche parmi la ou les poches (110, 210, 310, 400) comprend un matériau qui régule le flux d'air vers un élément chauffant inséré dans la poche.

9. Procédé de fabrication d'une couverture chauffante (100, 200, 300, 700, 800, 900) selon la revendication 1, comprenant les étapes suivantes :
fournir un corps (105, 205, 305) comprenant une première surface (120) pour la couverture chauffante ;
fournir une ou plusieurs poches (110, 210, 310, 400), chaque poche parmi la ou les poches étant conçue pour contenir un élément chauffant amovible respectif ; et
monter la ou les poches sur le corps ;
**caractérisé en ce que** la ou les poches sont conçues de telle sorte que ledit élément chauffant soit insérable de manière amovible dans la poche, la poche comprenant une première partie (405) qui s'étend depuis un bord de la poche et une seconde partie (410) qui s'étend depuis le bord opposé, l'élément chauffant étant insérable entre la première partie et la seconde partie, et la première partie et/ou la seconde partie pouvant être ajustées ou manipulées de sorte que l'élément chauffant soit situé sous la première partie et la seconde partie, et la première partie et/ou la seconde partie appliquant une force de compression sur l'élément chauffant.
